# EUROPEAN PATENT APPLICATION

(11) **EP 2 564 762 A1**
(43) Date of publication of application: **06.03.2013**
(21) Application number: 12005889.6
(22) Date of filing: 14.08.2012
(51) Int. Cl.: A61B 3/00, A61B 3/036, A61B 3/103, A61B 3/13, A61B 3/14, A61F 9/007

(54) **A microscope for ophtalmologic surgery**

(30) Priority: 29.08.2011 JP 2011185423
(71) Applicant: Kabushiki Kaisha Topcon, Tokyo 174-8580 (JP)
(72) Inventor: Sato, Toshiaki, Tokyo 174-8580 (JP); Takeda, Takanori, Tokyo 174-8580 (JP); Kitajima, Nobuaki, Tokyo 174-8580 (JP)
(74) Representative: Gagel, Roland

(57) **Abstract**

According to an embodiment, an attachment is attached to a main body part and has multiple bright points arranged in a ring. A calculation part calculates a first astigmatic axis angle based on an image obtained by using an imaging optical system to photograph the patient's eye while the multiple bright points are projected thereto. A storage stores the calculated first astigmatic axis angle and/or a second astigmatic axis angle measured before surgery. A mechanism changes the orientation of the main body part. A first input part inputs orientation information indicating the orientation of the main body part. A correction part corrects the first astigmatic axis angle and/or the second astigmatic axis angle based on the input orientation information. A display part displays the corrected first astigmatic axis angle and/or the corrected second astigmatic axis angle.

## Description

### [Field of the Invention]

The present invention relates to a microscope for ophthalmologic surgery.

### [Background of the Invention]

A microscope for ophthalmologic surgery, which can measure the astigmatic axis angles of a patient's eye and project the measurement results onto the patient's eye, is known (Refer to Japanese published unexamined application 2011-110172). The microscope for ophthalmologic surgery projects multiple bright points arranged in a circle onto the patient's eye, calculates the astigmatic axis angle based on the arrangement of these projection images, and is used for transplant surgeries of toric IOL (Intraeyepiece lens) for correcting astigmatism.

To effectively correct astigmatism, not only the astigmatic degree but also the astigmatic axis angle is important. Consequently, it is necessary to adjust the orientation of the lens with high accuracy when transplanting the toric IOL. The microscope for ophthalmologic surgery in Japanese published unexamined application 2011-110172 satisfies such needs. Furthermore, it is also advantageous in that the traditional method that directly marks the astigmatic axis angle on the patient's eye does not have to be followed.

### [Prior Art Documents]

### [Patent Documents]

[Patent Document 1] Japanese published unexamined application 2011-110172

### [Summary of the Invention]

### [Problem to be Solved by the Invention]

In transplant surgeries of toric IOLs, etc., reference is made not only to astigmatism information measured during surgery but also to measured values obtained before surgery. Moreover, because the operator reads the patient's medical chart before surgery, it is normal for the patient's astigmatism information to be in the operator's mind.

As an astigmatic axis angle, a prescribed coordinate axis (e.g., the horizontal axis) of a two-dimensional orthogonal coordinate system is set as 0° and an angle is defined in a prescribed direction (e.g., in the counterclockwise direction). Moreover, preoperative astigmatism measurement is performed in a sitting position using a keratometer or a refractometer, but during surgery, astigmatism measurement is performed in a recumbent (supine) position. As a result, the orientation of the coordinate systems differs between before surgery and during surgery, and even if the actual astigmatic axis angle is the same, the values expressed in these coordinate systems differ. When such a situation occurs, this may cause misunderstanding or confusion for the operator. Moreover, although the microscope for ophthalmologic surgery described in Japanese published unexamined application 2011-110172 is able to project the astigmatic axis angle to the patient's eye using multiple bright points, such differences in the orientation of the coordinate systems may result in information indicating an inaccurate direction being projected.

Moreover, during surgery, the orientation of the microscope differs depending on the position of the operator (i.e., on the parietal side, the right ear side, or the left ear side, etc. of the patient), and therefore, a similar situation occurs when measurements are performed multiple times by changing the orientation of the microscope.

This invention has been devised to resolve the above problems, and the objective thereof is to provide a microscope for ophthalmologic surgery that can present, with consistency, multiple astigmatic axis angles measured at different orientations.

### [Means for Solving the Problem]

In order to achieve the above objective, a microscope for ophthalmologic surgery according to Claim 1 comprises: an optical system comprising an imaging optical system that photographs a patient's eye; a main body part that stores at least part of the optical system; an attachment that is attached to the main body part and has multiple bright points arranged in a ring; a calculation part that calculates a first astigmatic axis angle based on an image obtained by using the imaging optical system to photograph the patient's eye while the multiple bright points are projected thereto; a storage that stores the calculated first astigmatic axis angle and/or a second astigmatic axis angle of the patient's eye measured before surgery; a mechanism that changes the orientation of the main body part to which the attachment is attached; a first input part that inputs orientation information indicating the orientation of the main body part; a correction part that corrects the first astigmatic axis angle and/or the second astigmatic axis angle based on the orientation information that has been input; and a display part that displays the corrected first astigmatic axis angle and/or the corrected second astigmatic axis angle.
An invention according to Claim 2 is a microscope for ophthalmologic surgery comprising: an optical system comprising an imaging optical system that photographs a patient's eye; a main body part that stores at least part of the optical system; an attachment that is attached to the main body part and has multiple bright points arranged in a ring; a calculation part that calculates a first astigmatic axis angle based on an image obtained by using the imaging optical system to photograph the patient's eye while the multiple bright points are projected thereto; a storage that stores the calculated first astigmatic axis angle and/or a second astigmatic axis angle of the patient's eye measured before surgery; a mechanism that changes the orientation of the main body part to which the attachment is attached; a first input part that inputs orientation information indicating the orientation of the main body part; a correction part that corrects the first astigmatic axis angle and/or the second astigmatic axis angle based on the orientation information that has been input; and a controller that identifies bright points corresponding to the corrected first astigmatic axis angle and/or the corrected second astigmatic axis angle from among the multiple bright points and turns on the identified bright points.
An invention according to Claim 3 is the microscope for ophthalmologic surgery according to Claim 1 or 2, wherein the first input part comprises a first operation part for inputting the orientation information.
An invention according to Claim 4 is the microscope for ophthalmologic surgery according to Claim 3, wherein the first input part comprises a presentation part that presents, as the orientation information, at least the parietal side and an ear side in a selectable manner using the first operation part, and when correcting the second astigmatic axis information, the correction part does not change the second astigmatic axis angle if the parietal side has been selected, and adds +90° or -90° to the second astigmatic axis angle if the ear side has been selected.
An invention according to Claim 5 is the microscope for ophthalmologic surgery according to Claim 1 or 2, wherein the first input part comprises a generation part that generates the orientation information by analyzing a photographed image of the patient's eye from the imaging optical system.
An invention according to Claim 6 is the microscope for ophthalmologic surgery according to any of Claims 1 through 5, wherein the microscope for ophthalmologic surgery further comprises a second operation part for inputting values of the astigmatic axis angle, and the correction part corrects the input value based on the orientation information.
An invention according to Claim 7 is the microscope for ophthalmologic surgery according to any of Claims 1 through 6, wherein the microscope for ophthalmologic surgery further comprises a second input part that inputs incision information indicating the length and direction of an incised wound on the anterior ocular segment of the patient's eye; and the correction part comprises an induced-astigmatism calculation part that calculates induced astigmatism based on the input incision information and either the first astigmatic axis angle or the second astigmatic axis angle, and performs the correction based on the calculated induced astigmatism and the orientation information.

### [Effect of the Invention]

The first mode of the microscope for ophthalmologic surgery according to the present invention is able to measure the astigmatic axis angle of a patient's eye by using the attachment attached to the main body part. Moreover, the storage of the microscope for ophthalmologic surgery stores the astigmatic axis angle (first astigmatic axis angle) calculated internally and/or the astigmatic axis angle (second astigmatic axis angle) of the patient's eye measured before surgery. Furthermore, the microscope for ophthalmologic surgery comprises a mechanism that changes the orientation of the main body part, to which the attachment is attached, and comprises a configuration that inputs the orientation information thereof. The microscope for ophthalmologic surgery is configured to then correct the first astigmatic axis angle and/or the second astigmatic axis angle based on the input orientation information and display the corrected astigmatic axis angle.

According to such a microscope for ophthalmologic surgery, it is possible to correct and display an astigmatic axis angle measured in the past in accordance with the orientation of the main body part. Consequently, it is possible to present, with consistency, multiple astigmatic axis angles measured at different orientations.

The second mode of the microscope for ophthalmologic surgery according to the present invention is able to measure the astigmatic axis angle of the patient's eye by using the attachment attached to the main body part. Moreover, the storage of the microscope for ophthalmologic surgery stores the astigmatic axis angle (first astigmatic axis angle) calculated internally and/or the astigmatic axis angle (second astigmatic axis angle) of the patient's eye measured before surgery. Furthermore, the microscope for ophthalmologic surgery comprises a mechanism that changes the orientation of the main body part, to which the attachment is attached, and comprises a configuration that inputs the orientation information thereof. The microscope for ophthalmologic surgery is configured to then correct the astigmatic axis angle based on the input orientation information and turn on bright points corresponding to the corrected astigmatic axis angle.

According to such a microscope for ophthalmologic surgery, it is possible to correct an astigmatic axis angle measured in the past in accordance with the orientation of the main body part and to project it to the patient's eye. Consequently, it is possible to present, with consistency, multiple astigmatic axis angles measured at different orientations.

### [Brief Description of the Drawings]

Fig. 1 is a schematic drawing showing an example of the exterior configuration of the microscope for ophthalmologic surgery pertaining to the embodiment.
Fig. 2 is a schematic drawing showing an example of the configuration of a projection image forming part in the microscope for ophthalmologic surgery pertaining to the embodiment.
Fig. 3 is a schematic drawing showing an example of the configuration of a projection image forming part in the microscope for ophthalmologic surgery pertaining to the embodiment.
Fig. 4 is a schematic drawing showing an example of the configuration of an optical system in the microscope for ophthalmologic surgery pertaining to the embodiment.
Fig. 5 is a schematic drawing showing an example of the configuration of an optical system in the microscope for ophthalmologic surgery pertaining to the embodiment.
Fig. 6 is a schematic block diagram showing an example of the configuration of a control system in the microscope for ophthalmologic surgery pertaining to the embodiment.
Fig. 7 is a schematic diagram showing an example of a display screen displayed by the microscope for ophthalmologic surgery pertaining to the embodiment.
Fig. 8 is a flowchart showing an example of an operation of the microscope for ophthalmologic surgery pertaining to the embodiment.
Fig. 9 is a flowchart showing an example of an operation of the microscope for ophthalmologic surgery pertaining to the embodiment.
Fig. 10 is a flowchart showing an example of an operation of the microscope for ophthalmologic surgery pertaining to the embodiment.

### [Mode for Carrying Out the Invention]

An example of an embodiment of the microscope for ophthalmologic surgery, as related to the present invention, will be explained in detail with reference to the diagrams.

### [Configuration]

### [Exterior configuration]

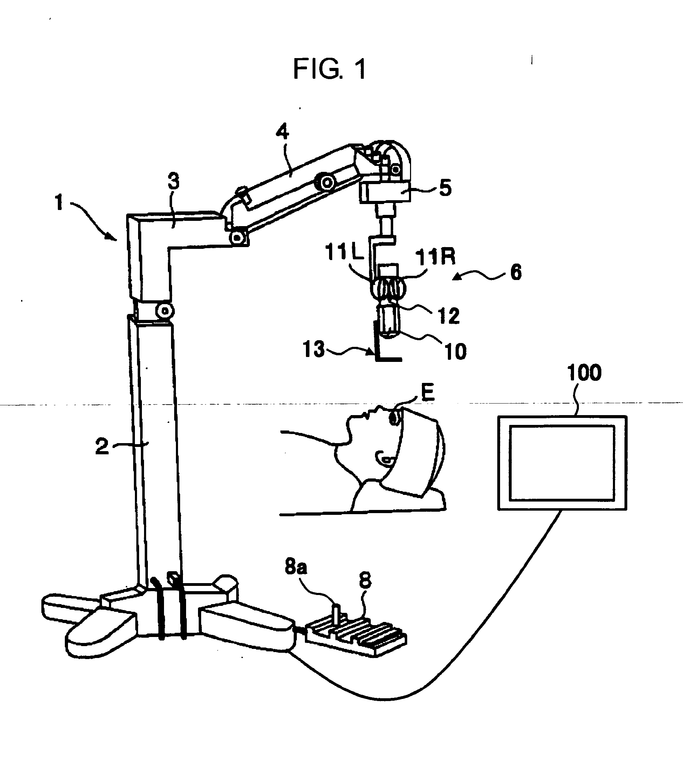

The exterior configuration of a microscope for ophthalmologic surgery 1, as related to the embodiment, will be explained with reference to Fig. 1. The microscope for ophthalmologic surgery 1 is composed of a pole 2, a first arm 3, a second arm 4, a driving device 5, a microscope 6, a foot switch 8 and a display part 100. It is possible to provide a computer between the pole 2 and the display part 100 and perform various types of information processing by using this computer.

Between the first arm 3 and the second arm 4, a mechanism for three-dimensionally moving the second arm 4 relative to the first arm 3 is provided. Moreover, at the attachment position of the microscope 6 relative to the arm extending downward from the driving device 5, it is also possible to provide a mechanism that makes the microscope 6 arbitrarily rotatable in the horizontal direction. These mechanisms and the driving device 5 correspond to mechanisms for changing the position and orientation of the microscope 6 (the main body part). Generally, the operator performs surgery while positioned at the parietal side or an ear side of the patient. The microscope 6 is arranged above the patient's eye E with the eyepiece lens facing the operator. The "orientation of the microscope 6" includes any concept indicating how the microscope 6 is arranged during surgery, such as the orientation of the eyepiece lens relative to the patient, the orientation of the eyepiece lens relative to the optical axis of the optical system (e.g., an objective lens), and the position of the operator relative to the patient etc.

The driving device 5 is composed of an actuator such as a motor. The driving device 5 moves the microscope 6 up and down as well as horizontally, depending on the operation using an operating lever 8a of the foot switch 8. This makes it possible to move the microscope 6 3-dimensionally.

Various optical systems, drive systems, etc. are stored in a lens tube part 10 of the microscope 6. An inverter part 12 is provided in the top part of the lens tube part 10. When an observation image of a patient's eye is obtained as a reversed image, the inverter part 12 converts the observation image to an erect image. A left-and-right pair of eyepiece parts 11 Land 11R is provided at the top part of the inverter part 12. An observer (such as an operator) can view the patient's eye with the both eyes by looking into the eyepiece parts 11L and 11R. The microscope 6 is an example of the "main body part."

The microscope for ophthalmologic surgery 1 has a projection image forming part 13. The projection image forming part 13 is removable from the microscope 6 and forms a specified projection image on the patient's eye E by projecting luminous flux onto the patient's eye E. The projection image forming part 13 is an example of an "attachment."

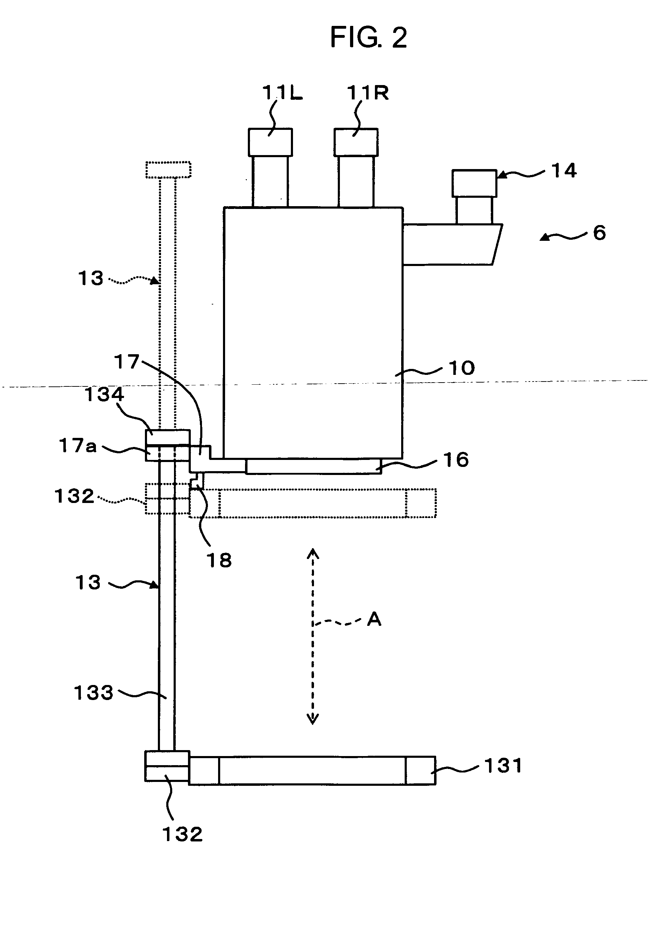

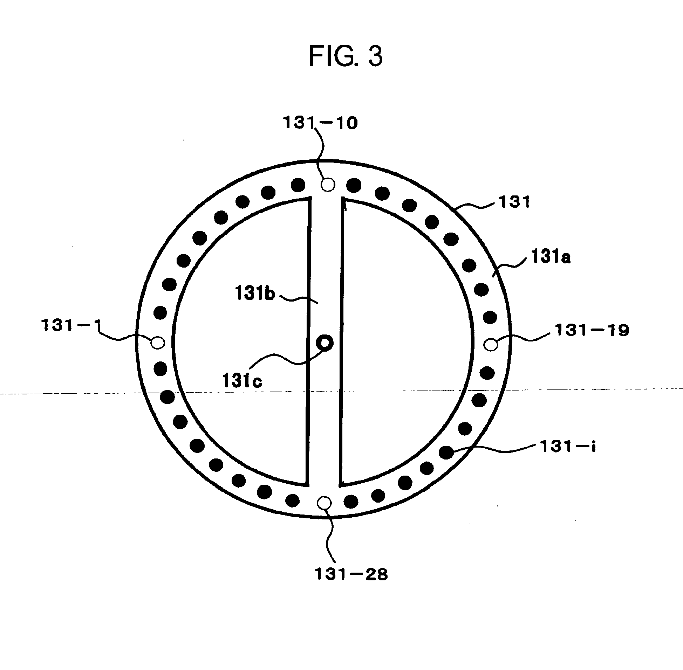

A configuration example of the projection image forming part 13 is shown in Fig. 2 and Fig. 3. The symbol 14 in Fig. 2 represents a photographing part. A TV camera 56, etc. that is discussed later is stored in the photographing part 14. Furthermore, Fig. 3 represents the configuration when a head part 131 of the projection image forming part 13 is viewed from the bottom (that is, from the side of the patient's eye E, in other words, from the side opposite to the lens tube part 10).

As indicated in Fig. 2 and Fig. 3, the head part 131 is a plate-like member. As indicated in Fig. 3, a toric periphery 131a and a fixation-light-source holder 131b, which is bridged in the direction of the diameter of the periphery 131a, are provided in the head part 131.

Multiple LEDs 131-i (i=1 to N) are provided on the bottom surface of the periphery 131a. A group of LED 131-i are placed in a ring. In this embodiment, 36 LEDs 131-i are provided at even intervals (N=36). That is, the group of LEDs 131-i are placed at intervals of 10 degrees with regard to the center position of the group of LEDs 131-i. In other words, when considering line segments that connect each LED 131-i and the center position, two line segments that relate to LED 131-i and the adjacent 131-(i+1) are crossed at the center position at an angle of 10°.

Each LED 131-i emits visible light. A group of LEDs 131-i can be composed such that all LEDs emit the same color and can also be composed to emit different colors. The latter case can be composed such that LEDs in the horizontal direction and the vertical direction in the group of LEDs 131-i output different colors from the LEDs in other directions. That is, it can be composed such that LEDs placed at astigmatic axis angles 0 degrees, 90 degrees, 180 degrees, and 270 degrees (LED 131-1, 131-10, 131-19, 131-28 accordingly) output luminous flux of different colors from other LEDs 131-i (i≠1, 10, 19, 28). For example, red LEDs can be used for LEDs 131-1, 131-10, 131-19, 131-28 along with green LEDs for other LEDs 131-i (i≠1, 10, 19, 28). As a result, the horizontal direction and the vertical direction of the astigmatic axes can be easily identified.

Furthermore, only a part of LED 131-1, 131-10, 131-19, and 131-28 can be set up to output luminous flux of different colors from other LEDs. For example, only LED 131i corresponding to a 0 degree angle can be composed to output different colors than other LEDs (i≠1).

In addition, it is not necessary that all of the LEDs 131-1, 131-10, 131-19, and 131-28 are composed to output luminous flux of the same color (red in the example above). For example, red LEDs can be used for LEDs 131-1 and 131-19 while white LEDs can be used for LEDs 131-10 and 131-28 and green LEDs can be used for other LEDs 131-i (i≠1, 10, 19, 28). As a result, the horizontal direction and the vertical direction of the astigmatic axes can be easily identified.

Moreover, instead of making the horizontal direction and vertical direction identifiable by an output color of a light source such as above, other configurations can have a similar effect. For example, changing the brightness of the output light can make the directions identifiable.

On the bottom surface of the fixation-light-source holder 131 b, a fixation target 131c for fixing the patient's eye E is provided. The fixation target 131c emits visible light. The output color of the fixation target 131c may be the same as that of the LEDs 131-i, but based on considerations for making it easy for the patient to recognize the fixation target 131c, it is desirable to make the output color of the fixation target 131c different from those of the LEDs 131-i. The fixation target 131 c is, for example, arranged at the central position of the LEDs 131-i that have been arranged in a ring.

The number of the fixation target 131 c is not limited to 1 but any number can be provided. Furthermore, the fixation target 131 c can be composed so as to be movable.

Moreover, the light source used for the head part does not have to be an LED and any device that can output light may be used. Furthermore, the multiple light sources placed in a ring do not have to be placed at even intervals. Further, because Fig. 3 is a diagram of the bottom surface, the placement order of the group of LEDs 131-i is set up in the opposite direction (reverse direction) from the direction set up for a general astigmatic axis. As a result, corneal reflection lights of luminous fluxes that re output from the group of LEDs 131-i (Purkinje's figure) are observed or photographed as the direction set up for a general astigmatic axis.

In addition, though 36 light sources are placed in a ring in this embodiment, the quantity is also arbitrary. It should be noted that when measuring the astigmatic axis angle of the patient's eye E based on the Purkinje's figure of luminous fluxes that are output by the group of LEDs 131-i, it is desirable that the quantity of light sources, which can ensure precision and accuracy of the measurements, are provided. When adopting a configuration that does not measure the stigmatic axis angles, there is no limit to the quantity of light sources in this sense.

Furthermore, depending on the precision required when the operator visually recognizes the orientation of the astigmatic axis angle and the principal meridian of toric IOL, the quantity of light sources can be properly set. For example, because light sources are placed at 10 degree intervals in this embodiment, the astigmatic axis angles can be presented in units of at least 10 degrees. In order to present the astigmatic axis angles, etc. with higher precision, the quantity of light sources, which corresponds to the precision (for example in units of 5 degrees, then 72 light sources), should be provided. The same can be said for lower precision cases.

Furthermore, though multiple light sources (the group of LEDs 131-i) are composed individually in this embodiment, this is not a restriction. For example, a display device (such as LCD (liquid-crystal display)) is provided on the bottom surface of the head part 131 and similar functionality can be obtained by displaying multiple bright points with this display device. In this case, each bright point corresponds to a light source.

An objective lens part 16 is provided at the bottom end of the lens tube part 10. An objective lens 15 (as stated below) is stored in the objective lens part 16. The supporting member 17 is provided in the vicinity of the objective lens part 16. The supporting member 17 is formed laterally from the objective lens part 16.

At the tip 17a of the supporting member 17, a vertically extending open hole is formed. An arm 133 is inserted into this open hole. The arm 133 is slidable inside the open hole. As a result, the arm 133 can be moved vertically (direction shown by the arrow A pointing at both sides in Fig. 2) with regard to the tip 17a. Here, the microscope 6 side is the top and the patient's eye E side is the bottom.

An anti-drop part 134 is provided at the top of the arm 133. The anti-drop part 134 is a plate-like member with a diameter bigger than the caliber of the open hole. As a result, the anti-drop part 134 prevents the arm 133 from falling from the tip 17a.

A head connecting part 132 is provided at the bottom end of the arm 133. The head connecting part 132 connects the head part 131 to the arm 133 such that the surface with LEDs 131-i face the bottom.

With such a configuration, the head part 131, the head connecting part 132, the arm 133, and the anti-drop part 134 (that is, the projection image forming part 13) can move freely and vertically with regard to the tip 17a. The projection image forming part 13 is moved by, for example, a user holding the arm 133, etc. Furthermore, by using a driving means such as a motor, the projection image forming part 13 can be composed such that it is moved electrically.

A connection hook 18 is provided on the bottom surface of the supporting member 17. The connection hook 18 is composed such that it can be engaged to an engaging part (not shown) of the projection image forming part 13. This engaging part is, for example, provided in the head connecting part 132. When the projection image forming part 13 is moved to the top, the engaging part engages the connection hook 18 to stop the vertical movement of the projection image forming part 13. This engagement relationship can be released by a specified operation (for example by pressing a specified button). The configurations of the connection hook 18 and the engaging part are optional.

With the above configuration, the group of LEDs 131-i is maintained such that they can move in the direction of the optical axis of the objective lens 15. Furthermore, the fixation target 131c is placed in the optical axis of the objective lens 15.

### [Configuration of the optical system]

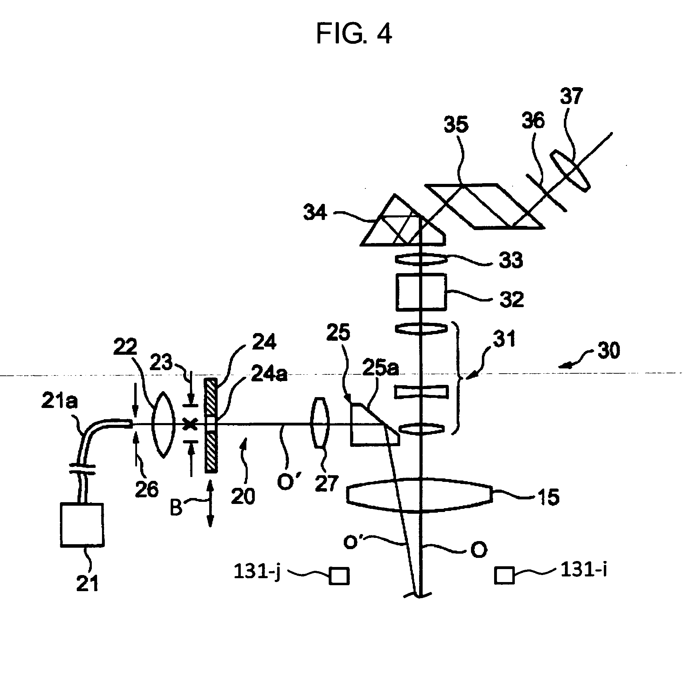

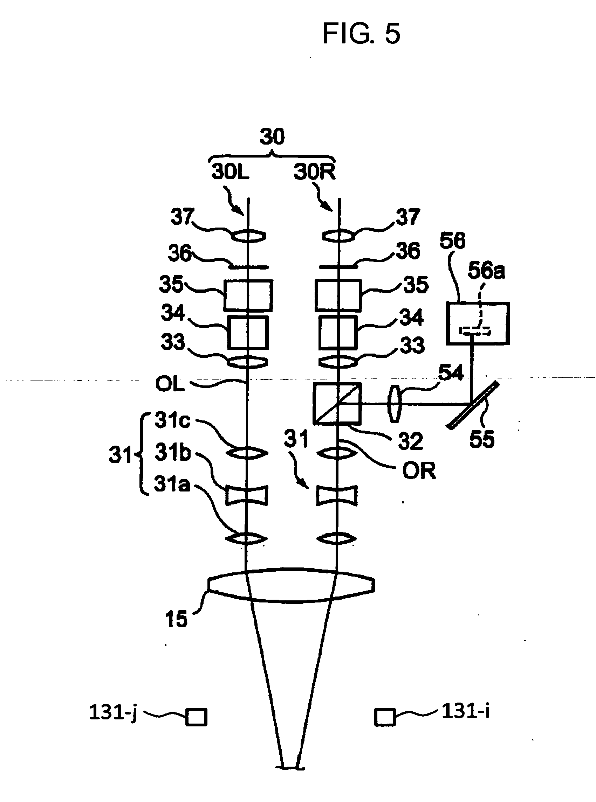

The optical system of the microscope for ophthalmologic surgery 1 will be explained next, with reference to Fig. 4 and Fig. 5. Here, Fig. 4 is a diagram, in which the optical system is viewed from the operator's left side. Moreover, Fig. 5 is a diagram, in which the optical system is viewed from the operator side. It should be noted that in addition to the configurations indicated in Fig. 4 and Fig. 5, an optical system (microscope for an assistant) for an assistant of the operator to observe the patient's eye E can be provided.

In this embodiment, directions such as top-and-bottom, left-and right, and front-and-back are directions viewed from the operator side unless otherwise specified. Moreover, with regards to the vertical direction, the direction from the objective lens 15 to the observation object (patient's eye E) is considered the lower direction while the opposite direction is considered the upper direction. Because patients generally go through an operation facing up, the top-and-bottom direction and the vertical direction become the same.

The group of LEDs 131-i is provided at the lower position of the objective lens 15 (that is, at the position between the objective lens 15 and the patient's eye E). Only LED 131-i and 131-j (i, j=1 to N, i≠j), which are positioned at both ends when viewed from the direction of this view point, are indicated in Fig. 4 and Fig. 5.

Moreover, a gap between the objective lens 15 and the patient's eye E means a gap between the position (height) of the objective lens 15 and the position (height) of the patient's eye E vertically (thus positions in the horizontal and front-and-back directions are not considered). Though the group of LEDs 131-i is placed in a ring, the diameter of this ring can be set arbitrarily as long as the size of an image of the light (bright point image) from the entire group of LEDs 131-i is projectable to the cornea of the patient's eye E.

An observation optical system 30 will be explained. A symmetrical pair of observation optical systems 30 is provided as indicated in Fig. 5. The observation optical system 30L on the left will be referred to as a left observation optical system and the observation optical system 30R will be referred to as a right observation optical system. A symbol OL indicates an optical axis (observation optical axis) of the left observation optical system 30L and a symbol OR indicates an optical axis (observation optical axis) of the right observation optical system 30R. The observation optical systems 30L and 30R on the left and right are arranged such that an optical axis O of the objective lens 15 is located in the middle of the optical axes OL and OR.

As before, each of the observation optical systems 30L and 30R on the left and right has a zoom lens system 31, a beam splitter 32 (only the right observation optical system 30R), an imaging lens 33, an image-erecting prism 34, a pupil-distance-adjusting prism 35, a field diaphragm 36, and an eyepiece lens 37.

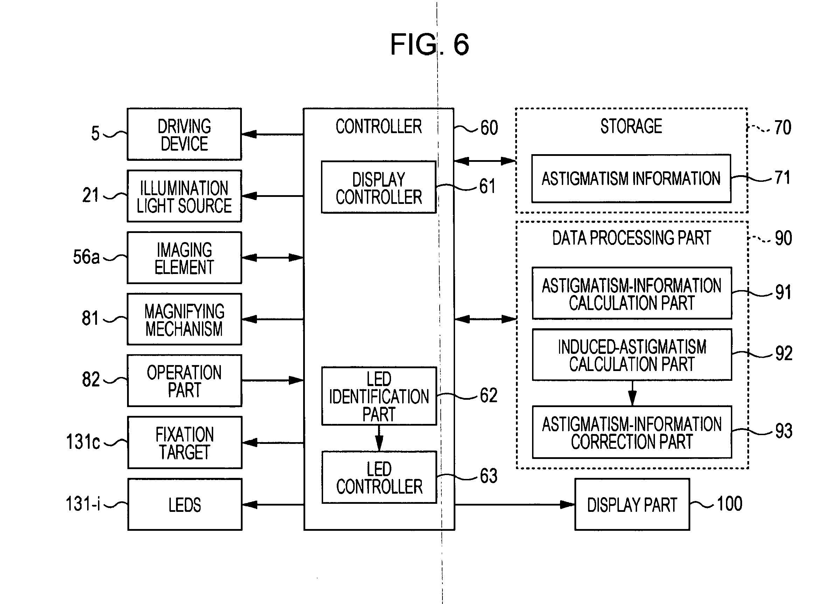

The zoom lens system 31 contains multiple zoom lenses 31a, 31b, and 31c. Each zoom lens 31a to 31c is movable in the direction of the observation optical axis OL (or the observation optical axis OR) by the magnifying mechanism 81 that will be mentioned later (refer to Fig. 6). As a result, the magnification is changed when observing or photographing the patient's eye E.

The beam splitter 32 of the right observation optical system 30R separates the part of the observation light that has been guided along the observation optical axis OR from the patient's eye E and leads to the TV camera imaging system. The TV camera imaging system comprises an imaging lens 54, a reflecting mirror 55, and a TV camera 56. The TV camera imaging system is stored in the photographing part 14. An optical element that is placed in the optical path from objective lens 15 to the TV camera 56 (imaging element 56a) is an example of an "imaging optical system."

The TV camera 56 is equipped with an imaging element 56a. The imaging element56a is, for example, composed of a CCD (Charge Coupled Devices) image sensor, CMOS (Complementary Metal Oxide Semiconductor) image sensor, etc. As the imaging element 56a, an element that has a two-dimensional light-receiving surface (that is, an area sensor) is used.

When using the microscope for ophthalmologic surgery 1, the light-receiving surface of the imaging element 56a is placed, for example, at an optically conjugated position with the corneal surface of the patient's eye E or at an optically conjugated position that is deep from the corneal apex by half of a corneal curvature radius.

The image-erecting prism 34 converts a reversed image to an erect image. The pupil-distance-adjusting prism 35 is an optical element for adjusting the distance between left and right observation lights in accordance with the pupil distance of the operator (the distance between left and right eyes). The field diaphragm 36 restricts the operator's field of vision by defilading the surrounding area in a cross-section of the observation light.

An illumination optical system 20 is explained next. As indicated in Fig. 4, the illumination optical system 20 comprises an illumination light source 21, an optical fiber 21a, an emission diaphragm 26, a condensing lens 22, an illumination field diaphragm 23, a slit plate 24, a collimator lens 27, and an illumination prism 25.

The illumination field diaphragm 23 is provided at a position that is optically conjugate to the front focal position of the objective lens 15. Furthermore, a slit hole 24a of the slit plate 24 is formed at the optically conjugate position against the front focal position.

The illumination light source 21 is provided outside the lens tube part 10 of the microscope 6. One end of the optical fiber 21 a is connected to the illumination light source 21. The other end of the optical fiber 21a is placed at a position facing the condensing lens 22 inside the lens tube part 10. Illumination light that is output from the illumination light source 21 enters the condensing lens 22 after being guided by the optical fiber 21 a.

The emission diaphragm 26 is provided at a position facing the exit end (fiber edge on the condensing lens 22 side) of the optical fiber 21a. The emission diaphragm 26 works such that some area of the exit end of the optical fiber 21a is covered. When the covered area is changed by the emission diaphragm 26, the exit area of the illumination light is changed. As a result, for example, the projection angle of the illumination light, that is the angle formed by the incident direction of the illumination light toward the patient's eye E and the optical axis O of the objective lens 15, can be changed.

The slit plate 24 is formed by a discoidal member with a light blocking effect. A transparent part that is composed of multiple slit holes 24a with shapes corresponding to the shape of the reflective surface 25a of the illumination prism 25 is provided on the slit plate 24. The slit plate 24 is moved in a direction orthogonal to an illumination optical axis O' (in the direction shown by the arrow B pointing at both sides in Fig. 4) by a drive mechanism (not drawn). As a result, the slit plate 24 is inserted into and removed from the illumination optical axis O'.

The collimator lens 27 turns the illumination light that has passed through the slit hole 24a into parallel luminous flux. The illumination light that has been turned into the parallel luminous flux is reflected by the reflective surface 25a of the illumination prism 25 and projected onto the patient's eye E via the objective lens 15. (Some of) the illumination light projected onto the patient's eye E is reflected by the cornea. The reflected light (sometimes referred to as observation light) of the illumination light by the patient's eye E enters the observation optical system 30 via the objective lens 15. Due to such a configuration, a magnified image of the patient's eye E becomes observable.

### [Configuration of the control system]

The control system of the microscope for ophthalmologic surgery 1 will be explained with reference to Fig. 6. Moreover, the control system is partially shown in Fig. 6. The drive mechanism for the slit plate 24, etc. is omitted.

### (Controller)

The control system of the microscope for ophthalmologic surgery 1 is composed around the controller 60. The controller 60 is provided at an arbitrary part of the microscope for ophthalmologic surgery 1. Furthermore, aside from the configuration indicated in Fig. 1, a computer and/or a circuit board can be provided and used as the controller 60. The controller 60 is composed of a microprocessor and a storage device similar to a normal computer.

The controller 60 controls each part of the microscope for ophthalmologic surgery 1. In particular, the controller 60 controls the driving device 5, the illumination light source 21, the magnifying mechanism 81, the group of LEDs 131-i, the fixation target 131c, the display part 100 etc. The driving device 5 moves the microscope 6 3-dimensionally after being controlled by the controller 60. The magnifying mechanism 81 moves each of the zoom lenses 31a, 31b, and 31 c of the zoom lens system 31 after being controlled by the controller 60. For example, a pulse motor is provided in the driving device 5 and the magnifying mechanism 81. The controller 60 controls the motion by sending pulse signals to each pulse motor.

The controller 60 is provided with a display controller 61, an LED identification part 62, and an LED controller 63.

### (Display controller)

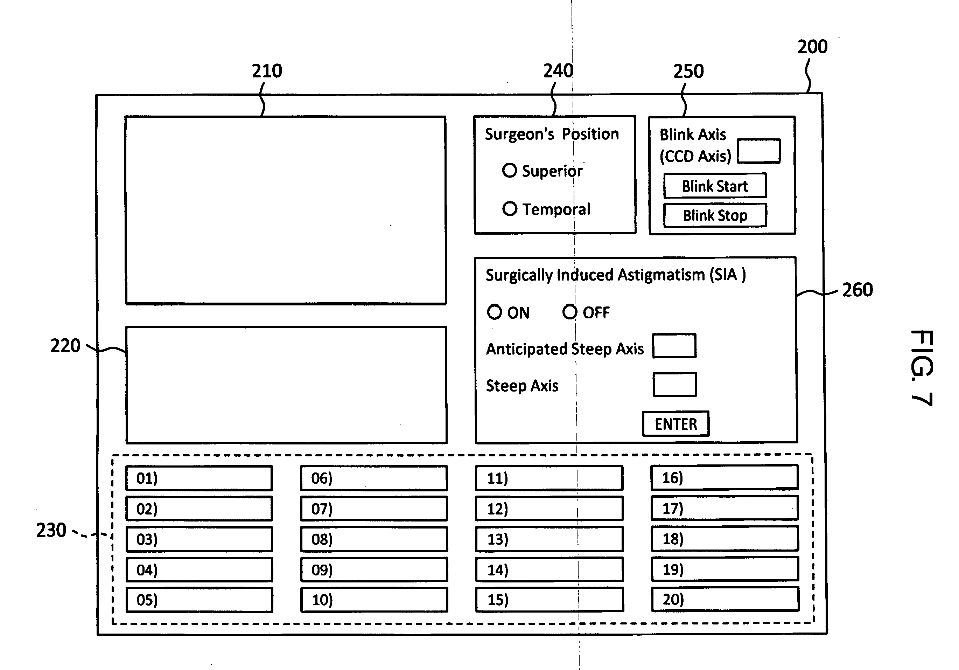

The display controller 61 displays various types of information on the display part 100. The various types of information include astigmatism information of the patient's eye E as well as the display screen 200 shown in Fig. 7 and information presented thereon, etc.

The display screen 200 is provided with an observation-image display part 210, an astigmatism-information display part 220, measured-value buttons 230, an operator-position input part 240, an astigmatic-axis angle input part 250, and an induced-astigmatism display part 260.

On the observation-image display part 210, photographed images of the patient's eye E, particularly live video, is displayed. On the astigmatism-information display part 220, astigmatism information of the patient's eye E, particularly the astigmatism axis angle measured by the microscope for ophthalmologic surgery 1 and the astigmatic axis angle corresponding to the measure-value button 230 selected by the user, is displayed.

On the measure-value buttons 230, a history of measured values of the astigmatic axis angle of the patient's eye E (i.e., measured values of the astigmatic axis angle from the microscope for ophthalmologic surgery 1, and measured values of the astigmatic axis angle obtained before surgery) is presented. When the user operates (clicks with a mouse) the measure-value button 230 presenting the desired measured value using the operation part 82, the display controller 61 displays the measured value presented on the measure-value button 230 on the astigmatism-information display part 220. Here, it is also possible to display the astigmatic degree acquired at the same time together with the astigmatic axis angle.

On the operator-position input part 240, information for inputting the operator's position (i.e., the orientation of the microscope 6) is presented. This input information is an example of the "orientation information". In this embodiment, the parietal side (superior) and the ear sides (temporal) are presented in a selectable manner. This selection operation is performed by designating (clicking with a mouse) the desired orientation information using the operation part 82. The operator-position input part 240 is an example of the "presentation part", and the operation part 82 is an example of the "first operation part".

The orientation information presented on the presentation part is not limited to this. For example, it is possible to provide a right ear side and a left ear side as the ear sides. The presentation part may comprise an input space allowing any orientation to be input, or may present multiple options for orientation information in a selectable manner.

Moreover, the "first input part" may be configured with the presentation part and the first operation part as in this embodiment, but may also be configured with only the first operation part used for inputting orientation information. As an example of such a case, it is possible to use an operation lever 8a of a footswitch 8 or a similar operation device as the first operation part. Generally, as long as the configuration allows orientation information indicating the orientation of the microscope 6 (main body part) to be input, the concrete configuration of the first input part may be of any type.

The astigmatic-axis angle input part 250 is provided with an input space for inputting values of the astigmatic axis angle. The user uses the operation part 82 (a keyboard) and inputs the values of a desired astigmatic axis angle. The operation part 82 is an example of the "second operation part". The Blink Start button is operated to cause the LED 131-i corresponding to the input value of the astigmatic axis angle to blink. The Blink Stop button is operated to stop the blinking of the LED 131-i.

The induced-astigmatism display part 260 displays an estimated value of the astigmatic axis angle (anticipated steep axis) of the patient's eye E that considers the astigmatism (induced astigmatism) that occurs when the anterior ocular segment of the patient's eye E is incised, as well as the value of the astigmatic axis angle (steep axis) that does not consider the induced astigmatism. By designating "ON" or "OFF", the user can select whether or not to perform processing to estimate the induced astigmatism.

The details will be described later, but the induced astigmatism is calculated based on incision information that is input using a second input part and indicates the length and direction of the incised wound. An example configuration of the second input part includes one comprising a graphic user interface (GUI) displayed on the display part 100 as well as the operation part 82. Moreover, another example of the second input part is one that inputs incision information that has been set in advance in accordance with the state of the patient's eye E or the surgical method or other attributes of the surgery from another device via a network. In this case, the second input part is a controller 60 comprising a communication function. Moreover, the data processing part 90 may be configured to analyze a photographed image of the patient's eye E and detect the length and direction of the incised wound. The configuration of the second input part is not limited to these examples, and as long as incision information can be input into the microscope for ophthalmologic surgery 1, the concrete configuration may be of any type.

### (LED identification part)

The LED identification part 62 identifies the LED 131-i associated with the predetermined astigmatic axis angle from among the LEDs 131-i. As described previously, each LED 131-i is associated with an astigmatic axis angle in advance. The LED identification part 62 stores in advance information on these associations. Then, when the LED identification part 62 receives an input of the predetermined astigmatic axis angle, by referring to this information, it identifies the LED 131-i associated with the astigmatic axis angle.

### (LED controller)

The LED controller 63 controls operations of the LEDs 131-i. In particular, the LED controller 63 turns on the LED 131-i identified by the LED identification part 62. This turning on includes not only continuous lighting up but also blinking and operational states (emission intensity, blinking speed, etc.) different from those of the other LEDs 131-j (wherein j ≠ i).

### (Storage)

The storage 70 stores various types of information. In particular, the storage 70 stores in advance astigmatism information 71. The astigmatism information 71 records measured values of the astigmatism from the microscope for ophthalmologic surgery 1 itself and/or measured values of the astigmatism obtained before surgery. The former corresponds to astigmatism parameters measured during surgery. The latter corresponds to astigmatism parameters measured by a device other than the microscope for ophthalmologic surgery 1, such as a keratometer or a refractometer. The astigmatism parameters include at least the astigmatic axis angle, and may also include the astigmatic degree, etc.

The astigmatism information 71 may record patient-specific information in an identifiable manner, but because surgery is generally performed on a single patient at a given time, measured values related to this patient may be recorded alone. Moreover, when operating on both the right and left eyes of a single patient, it is desirable for information on the left eye and information on the right eye to be recorded in the astigmatism information 71 in an identifiable manner. To perform identification such as that described above, prescribed attributes information (patient ID, left/right eye ID, etc.) should be associated with the measured values and recorded in the astigmatism information 71. Moreover, information indicating the date and/or time of measurement may also be associated with each measured value and recorded.

It is also possible to associate information indicating the arrangement relationship between the patient's eye E and the measurement device during measurement with the measured values. Examples of this arrangement relationship include information indicating the body posture (seated, supine, etc.) of the patient during measurement or the specific position (orientation) of the measurement device relative to the patient.

If multiple items of information are recorded in the astigmatism information 71, the user can use the operation part 82 to call up a desired item of information from among the information included in the astigmatism information 71. A GUI for doing so may be displayed on the display part 100.

### (Operation part)

The operation part 82 is used by an operator, etc. to operate the microscope for ophthalmologic surgery 1. The operation part 82 includes all types of hardware keys (buttons, switches, etc.) that are provided in a case, etc. of the microscope 6 as well as the foot switches 8. Furthermore, when a touch panel display and GUI are provided, various types of software keys displayed on these are included in the operation part 82. The operation part 82 may include pointing devices such as a mouse etc. and/or character input devices such as a keyboard etc.

### (Data processing part)

The data processing part 90 executes various types of data processing. The data processing part 90 is provided with an astigmatism-information calculation part 91, an induced-astigmatism calculation part 92, and an astigmatism-information correction part 93.

### (Astigmatism-information calculation part)

Based on photographed an image of the patient's eye E while the luminous fluxes from the LEDs 131-i are projected to the cornea, the astigmatism-information calculation part 91 calculates the astigmatic axis angle of the patient's eye E. This process includes a calculation process similar to that of a conventional keratometer, etc., and is executed based on the shape of the Purkinje's figure depicted in the photographed images (i.e., the arrangement of the multiple bright points).

### (Induced-astigmatism calculation part)

Based on the incision information (the length and direction of the incised wound) input by the second input part described previously as well as the astigmatic axis angle of the patient's eye E, the induced-astigmatism calculation part 92 calculates an estimated value of the induced astigmatism generated in the patient's eye E due to the surgery. Any known technology is applied for this calculation process. Examples of this known technology include the technology described in Japanese published unexamined application 2009-542360 as well as technology described in documents referred to therein.

### (Astigmatism-information correction part)

Based on the (estimated) induced astigmatism calculated by the induced-astigmatism calculation part 92 and the orientation information input into the operator-position input part 240, the astigmatism-information correction part 93 corrects the astigmatic axis angle of the patient's eye E. A configuration may be used in which the astigmatic degree is also corrected together with the astigmatic axis angle.

The induced-astigmatism calculation part 92 and the astigmatism-information correction part 93 of this embodiment constitute an example of the "correction part". On the other hand, it is also possible to apply a "correction part" configured without the induced-astigmatism calculation part 92. In this case, the astigmatism-information correction part 93 corrects the astigmatic axis angle of the patient's eye E based on the orientation information input into the operator-position input part 240.

Processes executed by the astigmatism-information correction part 93 are described. As described previously, preoperative astigmatism measurement is generally performed in a seated position. On the other hand, astigmatism measurement performed during surgery (i.e., astigmatism measurement using the microscope for ophthalmologic surgery 1) is generally performed in the supine position. Furthermore, in astigmatism measurement performed during surgery, the orientation of the microscope 6 and therefore also the orientation of the projection-image forming part 13 (LEDs 131-i) attached thereto are determined arbitrarily.

When the arrangement relationship between the patient and the measurement device differs in this way, even though the actual astigmatic axis angle of the patient's eye E does not change in practice, the measured value of the astigmatic axis angle in the first arrangement relationship and the measured value in the second arrangement relationship differ greatly.

For example, if expressing the astigmatic axis angle using the rightward direction in the frames of a photographed image of the patient's eye E as the standard direction (angle: 0°) and the counterclockwise direction as the positive direction, a 90° gap is generated between the measured value obtained using a keratometer, etc. in a seated position and the measured value obtained using the microscope for ophthalmologic surgery 1 with the operator positioned at an ear side. This gap is generated due to a difference in the standard direction caused by a difference in the arrangement relationship between the patient and the measurement device.

The astigmatism-information correction part 93 corrects these types of gaps. This correction may involve matching the standard direction of one measurement with the standard direction of the other measurement, or matching both relative to the different standard direction from those. The standard direction that acts as the standard for this matching can be set arbitrarily. Alternatively, a configuration may be used in which matching is performed constantly relative to a preset standard direction.

If measurement is performed while the operator is positioned on the parietal side, a 180° gap is generated between this measured value and the measured value obtained in the seated position, but because astigmatic axis angles that differ by 180° are viewed as the same, there is no need for correction.

If considering measurements from the right ear side and the left ear side separately, the symbol for the correction value (i.e., positive [+] or negative [-]) is determined in accordance with the definition of the positive direction of the angle. For example, if the rightward direction in the frame is defined as the standard direction and the counterclockwise direction is defined as the positive direction, when matching the measured value obtained from the right ear side with the measured value obtained in the seated position, it is necessary to rotate the standard direction by +90°, and the astigmatism-information correction part 93 therefore performs a correction to add -90° to the measured value θ of the right ear side (i.e., a correction to subtract 90° from the measured value θ).

Similarly, when matching the measured value obtained from the left ear side with the measured value obtained in the seated position, the astigmatism-information correction part 93 performs a correction to add +90° to the measured value of the left ear side. Moreover, when matching the measured value obtained in the seated position with the measured values of the right or left ear side, the opposite calculations of those described above are performed.

Moreover, when matching the measured values between an arbitrary first orientation and second orientation, correction values can be calculated in a similar manner based on the difference between the first orientation and the second orientation.

Corrections related to the induced astigmatism can be performed independently of the above corrections related to the orientation during measurement. That is, the gaps in the astigmatic axis angle caused by both are unrelated. Consequently, when matching a first measured value θ₁ with a second measured value θ₂, the astigmatism-information correction part 93 adds a correction value Δθ_{D} based on the difference in orientation between both measurements and a correction value Δθ₁ calculated by the induced-astigmatism calculation part 92 to the first measured value θ₁ to obtain the correction result θ1 + Δθ_{D}+ Δθ_{I}.

### [Operation]

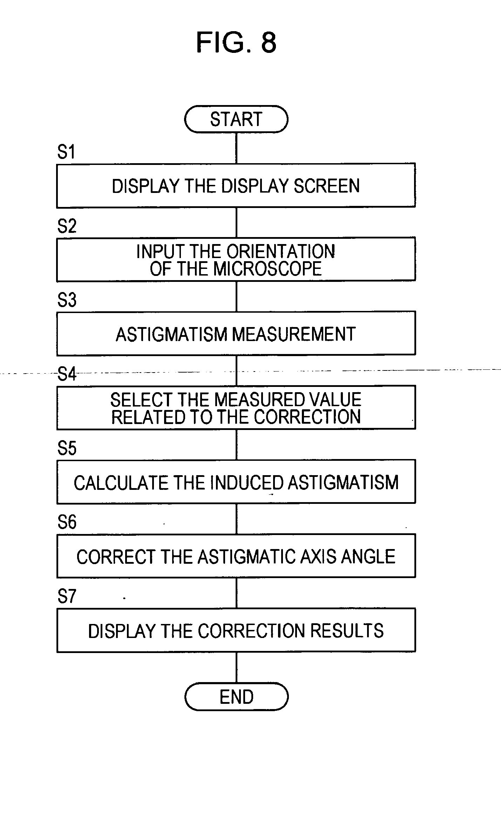

Operations of the microscope for ophthalmologic surgery 1 are described. Example operations of the microscope for ophthalmologic surgery 1 are shown in Fig. 8 through Fig. 10.

Fig. 8 is an example operation in a case in which the result of matching a new measured value with a past measured value is displayed. The new measured value is a measured value obtained during surgery, and the past measured value is a measured value obtained either before or during surgery. Here, the past measured value is a value measured before surgery in the seated position.

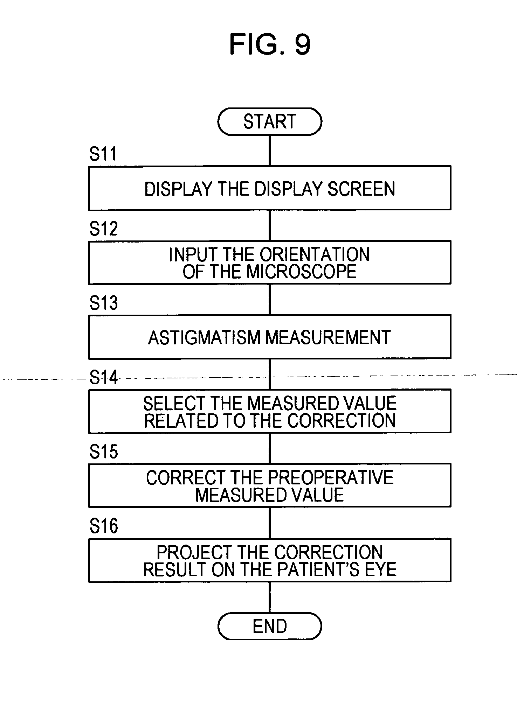

Fig. 9 is an example operation in a case in which a measured value obtained in the past is matched with the current orientation of the microscope 6 and projected to the patient's eye E.

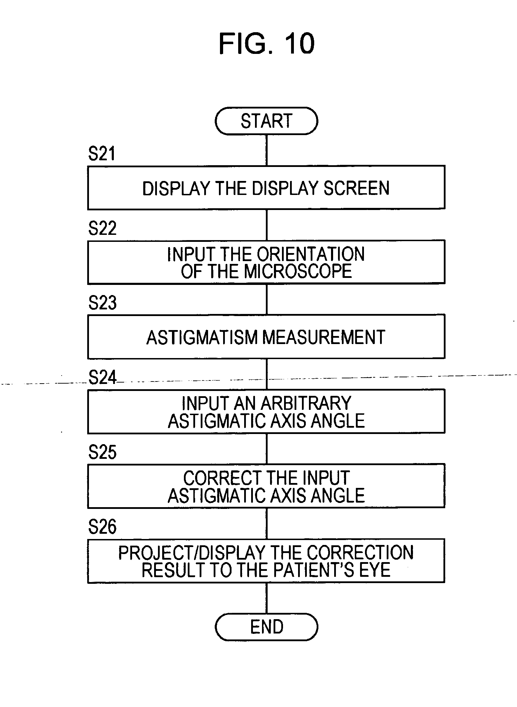

Fig. 10 is an example operation in a case in which the astigmatic axis angle input arbitrarily by the user is matched with the current orientation of the microscope 6. The result of this matching is displayed or projected.

### (First example operation: Fig. 8)

### (S1: Display the display screen)

The display controller 61 causes the display part 100 to display the display screen 200. On the measured-value buttons 230, measured values obtained in the past (i.e., before surgery) are listed. In this embodiment, the smaller of the numbers (01-20) presented on the measured-value buttons 230 are the newer measured values. These measured values are read out from the astigmatism information 71. Moreover, at this stage, for example, an input regarding whether or not to perform corrections based on the induced astigmatism is made in the induced-astigmatism display part 260 using the operation part 82. In this example operation, the relevant correction is to be performed.

### (S2: Input the orientation of the microscope)

The operator decides a standing position relative to the patient, who is lying in a supine position on the bed, decides the orientation of the microscope 6, and adjusts the position of the microscope 6 in a manner suitable for observing the patient's eye E. Then, the standing position of the operator (i.e., the orientation of the microscope 6) is input into the operator-position input part 240 of the display screen 200.

This input operation may be performed by the operator directly operating the operation part 82, or may be performed by an assistant or nurse who received instructions from the operator. If the orientation of the microscope 6 is changed, an input is made into the operator-position input part 240 each time. The person operating the display screen 200 is hereinafter referred to as the user.

### (S3: Astigmatism measurement)

The operator performs a prescribed operation in order to begin observation of the patient's eye E. The patient's eye E is photographed by a TV camera 56a and observation images (live video) are displayed on the observation-image display part 210. Moreover, the operator turns on the LEDs 131-i and the fixation target 131c at a desired timing and starts astigmatism measurement. Based on the photographed images obtained by the TV camera 56, the astigmatism-information calculation part 91 calculates the astigmatism parameters (particularly the astigmatic axis angle) of the patient's eye E.

The controller 60 records the calculated astigmatism parameters in the recorded information 71. Furthermore, the display controller 61 displays the calculated astigmatic axis angle on the measured-value button 230 of the number 01, and for each measured value displayed from before, the respective measured-value buttons 230 thereof are shifted down by one. Here, the mode in which measured values acquired during surgery are presented may differ from the mode in which measured values obtained in the past are presented (e.g., by changing the display color or changing the row or column in which they are displayed).

### (S4: Select the measured value related to the correction)

The user selectively operates the measured-value buttons 230 on which the measured value related to the correction of the measured values is presented. Here, a measured value acquired at least before surgery is selected. The configuration may be one in which the measured value acquired immediately before (i.e., the measured value acquired in step 3) is designated, or one in which this is not designated (in cases of designs in which this measured value is selected as a matter of course). The display controller 71 causes the astigmatism-information display part 220 to display the measured value selected by the user.

### (S5: Calculate the induced astigmatism)

Based on the incision information that is preliminarily input and stored in the storage 70 as well as the astigmatic axis angle acquired in step 3, the induced-astigmatism calculation part 92 calculates the induced astigmatism (the estimated value thereof; i.e., the correction value).

### (S6: Correct the astigmatic axis angle)

Based on the orientation of the microscope 6 input in step 2, (information indicating the arrangement relationship between the patient's eye E and the measurement device when the past measured value selected in step 4 was obtained,) and the induced astigmatism calculated in step 5, the astigmatism-information correction part 93 corrects the astigmatic axis angle acquired in step 3. As a result, the measured value acquired in step 3 is expressed in the coordinate system of the past measured value selected in step 4.

It should be noted that regarding the information indicating the above arrangement relationship in a past measurement, in cases of a design in which preoperative measured values are always measured in a seated position, it is not necessary to refer to this information.

### (S7: Display the correction results)

The display controller 61 displays the corrected astigmatic axis angle obtained in step 6 in the space to the right of the "Anticipated Steep Axis" of the induced-astigmatism display part 260. Moreover, the display controller 61 displays the value obtained by removing the component of the induced astigmatism from the corrected astigmatic axis angle obtained in step 6 in the space to the right of the "Steep Axis".

The operator is able to compare the measured value displayed on the induced-astigmatism display part 260 with the preoperative measured values displayed on the astigmatism-information display part 220. Because all of these measured values are expressed by the same coordinate system (the latter coordinate system), this comparison is meaningful. This concludes the description of this example operation.

### (Second example operation: Fig. 9)

### (S 11: Display the display screen)

As with the first example operation, the display screen 200 is displayed on the display part 100.

### (S12: Input the orientation of the microscope)

As with the first example operation, the orientation of the microscope 6 is input.

### (S13: Astigmatism measurement)

As with the first example operation, astigmatism measurement and accompanying processes are executed. It is also possible to execute the following processes before performing this astigmatism measurement.

### (S 14: Select the measured value related to the correction)

As with the first example operation, the measured value related to the correction is selected. In particular, a measured value acquired before surgery is designated.

### (S 15: Correct the preoperative measured value)

Based on the information indicating the orientation of the microscope 6 input in step 12 and the astigmatic axis angle measured before surgery selected in step 14 (as in the first example operation, the arrangement relationship between the patient's eye E and the measurement device during the preoperative measurement is also referred to if necessary), the astigmatism-information correction part 93 corrects the astigmatic axis angle. This correction result is obtained by matching the measured value of the preoperative astigmatic axis angle with the current orientation of the microscope 6.

### (S16: Project the correction result on the patient's eye)

The LED identification part 62 identifies the LED 131-i corresponding to the astigmatic axis angle corrected in step 15. Then, the LED controller 63 causes the identified LED 131-i to blink. The mode of control of the LED 131-i is not limited to blinking as long as the operational mode of the LED 131-i differs from that of the other LEDs 131-j (wherein j ≠ i).

As a result, the astigmatic axis angle obtained before surgery can be presented on the patient's eye E while matching the current orientation of the microscope 6. Moreover, it becomes possible to easily perform comparisons with the astigmatic axis angle measured during surgery. This concludes the description of this example operation.

### (Third example operation: Fig. 10)

### (S21: Display the display screen)

As with the first example operation, the display screen 200 is displayed on the display part 100.

### (S22: Input the orientation of the microscope)

As with the first example operation, the orientation of the microscope 6 is input.

### (S23: Astigmatism measurement)

As with the first example operation, astigmatism measurement and accompanying processes are executed. It should be noted that it is also possible to execute the following processes before performing this astigmatism measurement.

### (S24: Input an arbitrary astigmatic axis angle)

The user inputs the astigmatic axis angle desired by the operator into the astigmatic-axis angle input part 250 and clicks the "Blink Start" button at a timing desired by the operator. The astigmatic axis angle input here is one that is expressed based on a general coordinate system; in other words, one that is expressed in a coordinate system of the orientation during measurement in the sitting position.

### (S25: Correct the input astigmatic axis angle)

The astigmatism-information correction part 93 corrects the astigmatic axis angle input in step 24 based on the orientation of the microscope 6 input in step 22. The correction result is obtained by matching the value of the input astigmatic axis angle with the current orientation of the microscope 6.

### (S26: Project/display the correction result to the patient's eye)

The LED identification part 62 identifies the LED 131-i associated with the astigmatic axis angle corrected in step 25. Then, the LED controller 63 causes the identified LED 131-i to blink. Moreover, the display controller 61 causes the display screen 200 to display the astigmatic axis angle corrected in step 25. The correction result is displayed on, for example, the astigmatism-information display part 220.

As a result, it is possible to match any astigmatic axis angle input by the operator with the current orientation of the microscope 6 and present it on the patient's eye E. Moreover, it is possible to convert any astigmatic axis angle input by the operator into the current orientation of the microscope 6 and display it. Moreover, it becomes possible to easily perform comparisons with the astigmatic axis angle measured during surgery.

If incision information has already been input, it is also possible to perform the correction process of step 25 by considering the effect of induced astigmatism. This concludes the description of this example operation.

### [Effects]

The effects of the microscope for ophthalmologic surgery 1 are described.

Using the projection-image forming part 13 attached to the microscope 6, the microscope for ophthalmologic surgery 1 is able to measure the astigmatic axis angle of the patient's eye E. Moreover, the storage 70 of the microscope for ophthalmologic surgery 1 stores the astigmatism information 71, in which the astigmatic axis angle (first astigmatic axis angle) calculated internally and/or the astigmatism axis angle (second astigmatic axis angle) of the patient's eye E measured before surgery are recorded. Furthermore, the microscope for ophthalmologic surgery 1 comprises a mechanism that changes the orientation of the microscope 6, to which the projection-image forming part 13 is attached, and comprises a function that inputs orientation information indicating the orientation of the microscope 6. In addition, the microscope for ophthalmologic surgery 1 is configured to correct the first astigmatic axis angle and/or the second astigmatic axis angle based on the input orientation information and to display the corrected astigmatic axis angle.

According to this type of microscope for ophthalmologic surgery 1, it is possible to correct and display the astigmatic axis angle measured in the past in accordance with the orientation of the microscope 6. Consequently, it is possible to present, with consistency, multiple astigmatic axis angles measured at different orientations.

Moreover, the microscope for ophthalmologic surgery 1 is configured to identify and turn on the LED 131-i associated with the corrected astigmatic axis angle from among the LEDs 131-i based on the input orientation information. It should be noted that, as described previously, this "turning on" includes not only continuous lighting up but also blinking and operational states different from those of the other LEDs 131-j (wherein j ≠ i).

According to this type of microscope for ophthalmologic surgery 1, it is possible to correct an astigmatic axis angle measured in the past in accordance with the orientation of the microscope 6 and project it onto the patient's eye E. The operator is able to observe the patient's eye E with the bright points projected thereto. Consequently, according to the microscope for ophthalmologic surgery 1, it is possible to present, with consistency, multiple astigmatic axis angles measured at different orientations.

Moreover, according to the microscope for ophthalmologic surgery 1, it is possible to correct any astigmatic axis angle that has been input in accordance with the orientation of the microscope 6 and to project it to the patient's eye E, or to display the values thereof. Consequently, even for an astigmatic axis angle that has been input arbitrarily, it is possible to match it with the current orientation of the microscope 6 and present it with consistency.

Moreover, according to the microscope for ophthalmologic surgery 1, it is possible to perform more accurate corrections of the astigmatic axis angle by considering, in addition to the orientation of the microscope 6, the effect of induced astigmatism caused by an incision of the anterior ocular segment.

### [Modified examples]

The configuration described above is nothing more than an example. Persons who intend to implement the present invention may make any changes, omissions, or additions within the scope of the present invention.

For example, in the above embodiments, orientation information indicating the orientation of the microscope 6 is input manually, it is also possible to apply a configuration in which this information is input automatically. As a specific example, a generation part (not illustrated) that analyzes a photographed image of the patient's eye E and generates the orientation information is provided in the data processing part 90. The generation part detects the orientation of the eye in the photographed image. This detection process is executed by, for example, acquiring the luminance distribution of the photographed image, identifying image regions corresponding to the white of the eye based on the luminance distribution, and detecting the orientation of the eye based on the positions of the image regions in the frame of the photographed image.

In this process, because two image regions corresponding to the white of the eye are generally identified, if these image regions are arranged in the vertical direction in the frame, the determination result becomes the "ear side", and if they are arranged in the horizontal direction in the frame, the determination result becomes the "parietal side".

As another example, the orientation of the eye can be detected based on the position or shape of the border between the image regions of the white of the eye and the image region of the iris. Moreover, it is also possible to identify the borders between the patient's eye and the eyelids and to detect the orientation of the eye based on the positions or shapes of the borders. Furthermore, it is also possible to identify the inner corner and/or the outer corner of the eye based on the borders between the patient's eye and the eyelids and to detect the orientation of the eye based on the positions thereof.

### [Explanation of Symbols]

1 Microscope for ophthalmologic surgery 5 Driving device
13 Projection image forming part
20 Illumination optical system
21 Illumination light source
30 Observation optical system
56a Imaging element
60 Controller
61 Display controller
62 LED identification part
63 LED controller
70 Storage
71 Astigmatism information
81 Magnifying mechanism
82 Operation part
90 Data processing part
91 Astigmatism-information calculation part
92 Induced-astigmatism calculation part
93 Astigmatism-information correction part
131-i LED
131c fixation target
100 Display part
200 Display screen
210 Observation-image display part
220 Astigmatism-information display part
230 Measured-value buttons
240 Operator-position input part
250 Astigmatic-axis angle input part
260 Induced-astigmatism display part

## Claims

1. A microscope for ophthalmologic surgery comprising:
an optical system comprising an imaging optical system that photographs a patient's eye;
a main body part that stores at least part of the optical system;
an attachment that is attached to the main body part and has multiple bright points arranged in a ring;
a calculation part that calculates a first astigmatic axis angle based on an image obtained by using the imaging optical system to photograph the patient's eye while the multiple bright points are projected thereto;
a storage that stores the calculated first astigmatic axis angle and/or a second astigmatic axis angle of the patient's eye measured before surgery;
a mechanism that changes the orientation of the main body part to which the attachment is attached;
a first input part that inputs orientation information indicating the orientation of the main body part;
a correction part that corrects the first astigmatic axis angle and/or the second astigmatic axis angle based on the orientation information that has been input; and
a display part that displays the corrected first astigmatic axis angle and/or the corrected second astigmatic axis angle.

2. A microscope for ophthalmologic surgery comprising:
an optical system comprising an imaging optical system that photographs a patient's eye;
a main body part that stores at least part of the optical system;
an attachment that is attached to the main body part and has multiple bright points arranged in a ring;
a calculation part that calculates a first astigmatic axis angle based on an image obtained by using the imaging optical system to photograph the patient's eye while the multiple bright points are projected thereto;
a storage that stores the calculated first astigmatic axis angle and/or a second astigmatic axis angle of the patient's eye measured before surgery;
a mechanism that changes the orientation of the main body part to which the attachment is attached;
a first input part that inputs orientation information indicating the orientation of the main body part;
a correction part that corrects the first astigmatic axis angle and/or the second astigmatic axis angle based on the orientation information that has been input; and
a controller that identifies bright points corresponding to the corrected first astigmatic axis angle and/or the corrected second astigmatic axis angle from among the multiple bright points and turns on the identified bright points.

3. The microscope for ophthalmologic surgery according to Claim 1 or 2,
wherein the first input part comprises a first operation part for inputting the orientation information.

4. The microscope for ophthalmologic surgery according to Claim 3, wherein
the first input part comprises a presentation part that presents, as the orientation information, at least the parietal side and an ear side
in a selectable manner using the first operation part, and when correcting the second astigmatic axis information, the correction part does not change the second astigmatic axis angle if the parietal side has been selected, and adds +90° or -90° to the second astigmatic axis angle if the ear side has been selected.

5. The microscope for ophthalmologic surgery according to Claim 1 or 2, wherein the first input part comprises a generation part that generates the orientation information by analyzing a photographed image of the patient's eye from the imaging optical system.

6. The microscope for ophthalmologic surgery according to any of Claims 1 through 5, wherein
the microscope for ophthalmologic surgery further comprises a second operation part for inputting values of the astigmatic axis angle, and
the correction part corrects the input value based on the orientation information.

7. The microscope for ophthalmologic surgery according to any of Claims 1 through 6, wherein
the microscope for ophthalmologic surgery further comprises a second input part that inputs incision information indicating the length and direction of an incised wound on the anterior ocular segment of the patient's eye, and
the correction part comprises an induced-astigmatism calculation part that calculates induced astigmatism based on the input incision information and either the first astigmatic axis angle or the second astigmatic axis angle, and performs the correction based on the calculated induced astigmatism and the orientation information.
